# EUROPEAN PATENT SPECIFICATION

(11) **EP 2 155 318 B1**
(45) Date of publication and mention of the grant of the patent: **06.03.2013**
(21) Application number: 07748134.9
(22) Date of filing: 15.05.2007
(51) Int. Cl.: A61N 1/05, A61N 1/02

(54) **MEDICAL IMPLANTABLE LEAD WITH FLEXIBLE SEGMENTED SLEEVE**
MEDIZINISCHE IMPLANTIERBARE LEITUNG MIT FLEXIBLER SEGMENTIERTER HÜLLE
CONDUCTEUR MÉDICAL IMPLANTABLE POURVU D'UN MANCHON SEGMENTÉ SOUPLE

(43) Date of publication of application: 24.02.2010
(73) Proprietor: St. Jude Medical AB, 175 84 Järfälla (SE)
(72) Inventor: HILL, Rolf, S-175 55 Järfälla (SE); STEGFELDT, Olof, S-138 57 Älta (SE); BARLOV, Armin, S-177 55 Järfälla (SE)
(74) Representative: Sjölander, Henrik
(86) International application number: PCT/SE2007/000470
(87) International publication number: WO 2008/140364

(56) References cited:
- EP-A1- 0 779 080
- EP-A1- 0 779 080
- WO-A1-99/15229
- DE-A1- 3 300 050
- US-A- 3 397 699
- US-A- 4 419 819
- US-A- 4 576 162
- US-A- 5 344 439
- US-A1- 2002 007 204
- US-A1- 2004 059 404
- US-A1- 2007 050 003
- US-A1- 2007 073 370

## Description

The invention relates to a medical implantable lead, which is adapted to be attached with a distal end to tissue inside a human or animal body and which comprises a flexible sleeve in the distal end which protrudes a distance in an axial direction from the rest of the lead, wherein when in a desired position, a force acting in an axial direction on the flexible sleeve will bring the sleeve to be compressed in the axial direction such that the surface area of the distal end will be enlarged.

### Background of the invention

Medical implantable leads of various kinds and for various applications, e.g. for monitoring and controlling the heart in a human or animal body by means of a pacemaker, tend to become smaller and smaller in cross section. A medical implantable lead for pacemaker applications, for example, can have a diameter of less than 2 mm. This is advantageous in one aspect, since then the lead will be more flexible and take up less space. However, there are also risks with leads having a too small cross sectional dimension. In many cases the leads are namely adapted to be mounted to an organ inside the body, e.g. a heart wall, with its end surface abutting the organ and held by means of for example a helix, which is screwed into the organ. If the cross sectional dimension of the lead is too small in relation to its stiffness, it is a risk that the lead tip will perforate the organ during mounting of the lead and cause bleeding. This risk exists on the one hand when the distal end is pressed against the organ but before the lead is actually attached to it. In case the lead is provided with a rotatable helix for attaching to the organ, this risk also exists when the helix is screwed into the organ such that the distal end is drawn into the tissue by means of the rotating action of the helix. When the lead is attached to e.g. a heart, which performs large movements during function, there is also a risk that the heart wall will be perforated or injured during the course of a longer period of time when the lead is attached, due to abrasion or the like, if the lead is made with a too small cross sectional dimension in relation to its stiffness.

From US 2007/0050003 A1, is known a medical implantable lead, which in one embodiment (Figs 7A-C) is provided with an elastic sleeve in a distal end which protrudes a distance beyond a distal end of the rest of the lead and which, when abutting the distal end against tissue or attaching the lead to the tissue by means of screwing a helix into the tissue, will buckle and expand to a diameter that is larger than the diameter of the distal end of the lead itself. However, there are several disadvantages with a medical implantable lead of this kind. To accomplish buckling of the sleeve, it is required a rather large force having to effect that, when the lead is mounted with its distal end in abutment against tissue, the sleeve will affect the lead with a force striving to push the lead from and disengage the helix from the tissue. This is not satisfactory since it may lead to that the helix might slip out of engagement with the tissue during long term usage due to movements in the tissue, e.g. a heart. To alleviate this effect, one possible solution would be to make the sleeve extremely elastic and flexible, but this would also have to effect that the contribution of the sleeve for preventing perforation into the tissue, would be reduced correspondingly. It would also have to effect that the risk for unintentional buckling of the sleeve, and hence an increased lead tip area, when introducing the lead through e.g. a narrow vein will increase, which might make it impossible to insert the lead.

EP 0 779 080 discloses a medical implantable lead including extendable tines for fixation to tissue. A flexible sleeve is arranged at a distal end of the lead and the tines are formed when pushing the sleeve in a proximal direction. The tines are formed at a distance from the outermost distal part of the lead intended to abut a wall.

US 3 397 699 discloses a catheter to be used for drainage of body cavities. The catheter includes a tube having apertures, for communication through its walls, at its distal end. Proximally of the apertures, the walls of the tube are provided with longitudinal slits and scored or indented at desired fold lines so as to form wings that are capable of folding outwardly. The wings constitute retaining means.

### Summary of the invention

It is an object of the invention to provide an improved medical implantable lead by which the risk of perforation into tissue is eliminated or reduced and yet can ensure secure attachment to tissue. At least this object is achieved by a medical implantable lead according to claim 1, which defines the invention.

The basis of the invention is the insight that the above object may be achieved by providing the medical implantable lead with means for varying the surface area of the distal end of the lead, such that the surface area has a minimum when introducing the lead into the body. Once the lead is localised inside the body, the surface area of the distal end can be enlarged when abutting the distal end against an organ or other tissue.

According to the invention, the enlargement of the surface area is accomplished by mechanically folding of material portions at the distal end. More precisely, the distal end of the lead comprises a flexible sleeve, of e.g. silicone plastics, which protrudes a distance beyond the rest of the medical implantable lead. The outermost, distal end of the flexible sleeve is unbroken and hold together as a ring segment, whereas it is provided with slots in the axial direction of the lead, from the unbroken ring segment and a distance in the proximal direction, such that flexible segments are formed in the sleeve. When applying an axial force on the sleeve in the axial direction towards the proximal end, the flexible segments will be folded outwards and increases in this way the surface area of the lead end. The axial force is applied by letting a rotatable helix engage with some kind of engagement formation on the ring segment, such as an engagement post on the inside of the ring segment or an aperture in a disk positioned over the distal end of the ring segment. The latter requires, as in the hereinafter described and illustrated embodiment, that the helix is rotatable from the proximal end of the lead, by any suitable means known in the art, but is not displaceable in the axial direction of the lead during rotation. The displaceable arrangement of the helix is normally done to withdraw the helix into a tubular header such that the helix is accommodated in the header to prevent it from penetrating and causing damage during introduction into the body. In this case this is not necessary since the flexible sleeve will cover the helix during introduction.

Normally, at least four flexible segments are required for performing the folding of the flexible segments without having to use an excessive force to accomplish the folding of the sleeve. The length of the slotted portion of the sleeve is preferably from the ring segment to the position of the distal end of the rest of the lead and can suitably have a length of about 1,5-2 mm.

To facilitate folding of the sleeve segments, they can, as in a hereinafter described and illustrated embodiment, be provided with one or more grooves around the circumference of the sleeve on the side of the sleeve towards which the sleeve segments will be folded. More precisely, on the inside of the sleeve in the middle of each sleeve segment and preferably also on the outside in the respective ends of the segments, wherein the innermost groove is located at the position of the distal end of the rest of the lead.

It is preferred if the sleeve in the folded and mounted state, does not press outwards in the axial direction with a force against the tissue, which might lead to that the attachment to the organ in the course of time might become disconnected. This is achieved by the helix being in engagement with an engagement formation on the ring segment, such as a post on the inside of the unbroken ring or an aperture in a disk. Additionally, a circumferential groove may be provided, which does not divide each segment into two equal parts but one part is longer than the other such that a dead centre locking is achieved of the flexible segments in the folded position. It is also possible to make the sleeve in a preformed folded position and let the helix stretch out the sleeve during insertion by means of engagement with an engagement formation. When attaching the lead to tissue by rotating the helix, the sleeve will be folded to the preformed shape.

### Brief description of the drawings

An embodiment of the invention will now be described by way of example by reference to the accompanying drawings, in which:
- Fig 1: is a perspective view of a distal end of a medical implantable lead in an introducing state;
- Fig 2: is a perspective view of the lead in fig 1 in a partly compressed state when the sleeve segments are being folded outwards;
- Fig 3: is a perspective view of the lead according to figs 2 and 3 in a mounting state with the flexible segments folded outwards;
- Fig 4: is a longitudinal section through the distal end of the lead in the introducing state;
- Fig 5: is a longitudinal section through the distal end of the lead being introduced through a vein;
- Fig 6: is a longitudinal section according to fig 5 when the lead is being attached to a heart wall and the flexible segments folded outwards;
- Fig 7: is a longitudinal section of the lead in a mounted state; and
- Fig 8: is a longitudinal section through the lead in an extracting state when being extracted out from a body through a vein and the sleeve segments are folded forward.

### Detailed description of one embodiment of the invention

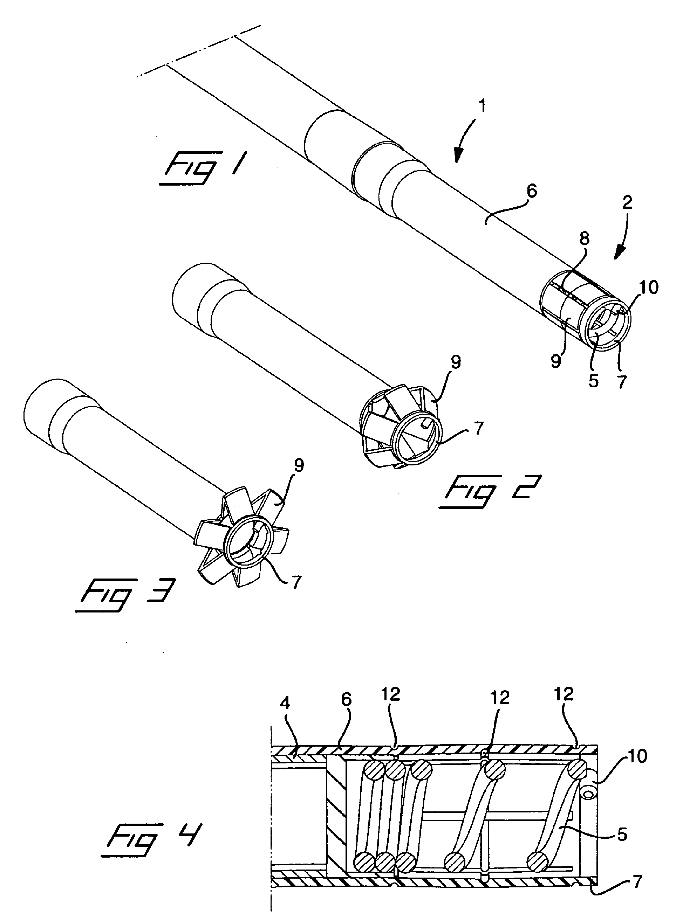

In the drawings is illustrated one embodiment of the invention in form of a medical implantable lead adapted to be used in connection with a pacemaker or with an implantable cardioverter defibrillator. Accordingly, the lead comprises a considerably long electrical lead 1, of which only a distal end is shown in the drawings. A pacemaker is adapted to be connected to a proximal end of the lead, whereas a distal end 2 is adapted to be attached to a heart wall 3. As is best seen in figs 4-8, the lead is, in a distal end, provided with a rigid tubular header 4 of metal or plastics, in the end of which a helix 5 is rotatably arranged. The helix can be rotated from the proximal end of the lead, by suitable means known in the art but not shown in the drawings, such as a stylet or a coil. On the outside of the header 4 the lead is provided with a sleeve 6 of a resilient material, which is extended a distance beyond the distal end of the header and covers the helix 5 completely in an introducing state, as is shown in fig 1, 4 and 5.

The sleeve is formed with an unbroken ring segment 7 in its distal end and axial slots 8, which extends from the unbroken ring segment 7 and a distance inwards, preferably to the distal end of the rest of the lead. In this way flexible segments 9 are formed between adjacent slots 8. On the inner side, the unbroken ring segment 7 is provided with an engagement means in form of a post 10, which is in engagement with the helix winding 5.

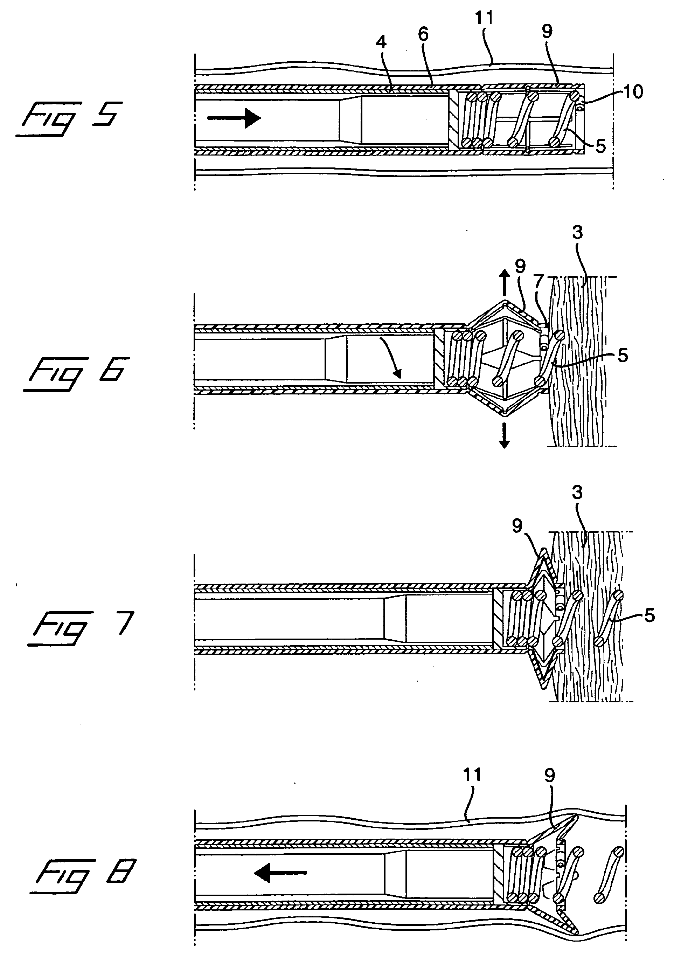

When implanting the lead into a human or animal body, e.g. by introducing it through a vein 11, as is illustrated in fig 5, the lead is in the introducing state as is also shown in fig 1. In this state the sleeve 6 covers the helix 5 and prevents it from penetration into tissue. When the lead has been introduced into the body and the distal end of the lead is bearing against tissue 3, at a location to be mounted in, the helix is caused to rotate by means of a rotary transmitting means, such as a stylet or a coil, by means of which a rotary movement can be transmitted from a proximal end to the distal end of the lead. When rotating the helix 5, its tip will penetrate into the tissue 3 at the same time as the unbroken ring segment 7 will be displaced towards the header 4 due to the engagement of the post 10 with the helix winding 5, as is illustrated in fig 6. This will bring the flexible segments 9 to fold outwards and when the unbroken ring segment 7 has reached its inner end position, as is illustrated in fig 7, the flexible segments 9 will be folded and oriented in a substantially radial direction around the periphery of the lead such that the surface area of the distal end of the lead is considerable increased in relation to the mounting state.

To facilitate folding of the flexible segments, these are provided with grooves 12 in a circumferential direction of the lead in the areas where the segments 9 are folded, i.e. in each end of the segments 9 as well as in the middle of each segment. The grooves 12 are best seen in fig 4 and, as can be seen, the grooves are formed on the side towards which it is to be folded, i.e. on the outside at the ends of the segments and on the inside in the middle of the segments.

In fig 8 is illustrated a situation in which the lead are to be retracted from inside the body. This situation may occur several years after implanting into the body and by that time, the distal end can be more or less overgrown by tissue such that folding back of the sleeve 6 to the introducing state in fig 1 may be difficult or even impossible. However, thanks to the highly flexible characteristics of the sleeve, the flexible segments can be "double folded", as is illustrated in fig 4, when the lead is being extracted and met with resistance from e.g. the inner walls of a vein 11. In this way the extraction will be facilitated.

## Claims

1. A medical implantable lead, which is adapted to be attached with a distal end (2) to tissue (3) inside a human or animal body, said lead comprises a rotatable helix (5) in the distal end for enabling attachment of the lead to the tissue by screwing the helix into the tissue and a flexible sleeve (6) in the distal end which protrudes a distance in an axial direction from the rest of the lead, wherein the flexible sleeve (6) is formed with axial slots (8) from an unbroken ring segment (7) in an area at an outermost distal edge of the sleeve and a distance in the proximal direction such that flexible segments (9) are formed in the sleeve, wherein when in a desired position, a force acting in an axial direction on the flexible sleeve will bring the sleeve to be compressed in the axial direction by folding outwards of the segments such that the surface area of the distal end will be enlarged, **characterized in that** said sleeve (6) comprises an engagement formation (10) on the distal ring segment (7), such that the engagement formation is in engagement with the helix and when screwing the helix into the tissue, the sleeve will be compressed by displacement of the ring segment in a proximal direction.

2. A medical implantable lead according to claim 1, **characterized in that** each flexible segment (9) is formed with a circumferential groove (12) on an inner side at an intermediate portion of the segment which will act as a hinge, to facilitate folding of the segments.

3. A medical implantable lead according to claim 2, **characterized in that** each flexible segment (9) is formed with a circumferential groove (12) on an outer side at each end of the segment which will act as a hinge, to facilitate folding of the segments.

4. A medical implantable lead according to claim 3, **characterized in that** the innermost circumferential groove (12) of each flexible segment (9) is positioned at the distal end of the rest of the lead.

5. A medical implantable lead according to any one of the preceding claims, **characterized in that** the engagement formation is in form of a post (10) on the inside of the ring segment (7).

6. A medical implantable lead according to any one of claims 1-4, **characterized in that** the engagement formation (10) is in form of an aperture in a disk on the ring segment (7).

7. A medical implantable lead according to any of the preceding claims, **characterized in that** the flexible sleeve (6) is provided with at least four flexible segments (9).

8. A medical implantable lead according to any of the preceding claims, **characterized in that** it also has an extracting state in which the outward folded segments (9) are bent in the distal direction, such that the surface area of the distal end is reduced in relation to the mounting state to facilitate extraction of the medical implantable lead from the human or animal body.

## Patentansprüche

1. Medizinische implantierbare Leitung, die ausgelegt ist, mit einem distalen Ende (2) an Gewebe (3) innerhalb eines menschlichen oder tierischen Körpers angebracht zu werden, wobei die Leitung eine drehbare Wendel (5) an dem distalen Ende, um eine Anbringung der Leitung an dem Gewebe durch Einschrauben der Wendel in das Gewebe zu ermöglichen, und eine flexible Hülle (6) an dem distalen Ende umfasst, welche ein Stück in einer axialen Richtung von dem Rest der Leitung vorsteht, wobei die flexible Hülle (6) mit axialen Schlitzen (8) aus einem ununterbrochenen Ringsegment (7) in einem Bereich an der äußersten distalen Kante der Hülle und einem Abstand in der proximalen Richtung gebildet ist, derart, dass flexible Segmente (9) in der Hülle gebildet werden, wobei dann, wenn sie in einer gewünschten Position ist, eine Kraft, die in einer axialen Richtung auf die flexible Hülle wirkt, die Hülle durch Falten der Segmente nach außen in einen in der axialen Richtung gestauchten Zustand bringt, derart, dass der Oberflächenbereich des distalen Endes vergrößert wird, **dadurch gekennzeichnet, dass** die Hülle (6) eine Eingriffsanordnung (10) an dem distalen Ringsegment (7) umfasst, derart, dass die Eingriffsanordnung in Eingriff mit der Wendel ist, und dass dann, wenn die Wendel in das Gewebe eingeschraubt wird, die Hülle durch einen Versatz des Ringsegments in einer proximalen Richtung gestaucht wird.

2. Medizinische implantierbare Leitung nach Anspruch 1, **dadurch gekennzeichnet, dass** jedes flexible Segment (9) mit einer Umfangsnut (12) an einer Innenseite an einer Zwischenposition des Segments gebildet ist, welche als ein Gelenk wirkt, um ein Falten der Segmente zu erleichtern.

3. Medizinische implantierbare Leitung nach Anspruch 2, **dadurch gekennzeichnet, dass** jedes flexible Segment (9) mit einer Umfangsnut (12) an einer Außenseite an jedem Ende des Segments gebildet ist, welche als ein Gelenk wirkt, um ein Falten der Segmente zu erleichtern.

4. Medizinische implantierbare Leitung nach Anspruch 3, **dadurch gekennzeichnet, dass** die innerste Umfangsnut (12) jedes flexiblen Segments (9) an dem distalen Ende des Rests der Leitung positioniert ist.

5. Medizinische implantierbare Leitung nach einem der voranstehenden Ansprüche, **dadurch gekennzeichnet, dass** die Eingriffsanordnung in der Form eines Stabs (10) an der Innenseite des Ringsegments (7) ist.

6. Medizinische implantierbare Leitung nach einem der Ansprüche 1-4, **dadurch gekennzeichnet, dass** die Eingriffsanordnung (10) in der Form einer Öffnung in einer Scheibe an dem Ringsegment (7) ist.

7. Medizinische implantierbare Leitung nach einem der voranstehenden Ansprüche, **dadurch gekennzeichnet, dass** die flexible Hülle (6) mit zumindest vier flexiblen Segmenten (9) ausgestattet ist.

8. Medizinische implantierbare Leitung nach einem der voranstehenden Ansprüche, **dadurch gekennzeichnet, dass** sie ferner einen Extraktionszustand aufweist, in welchem die nach außen gefalteten Segmente (9) in der distalen Richtung gebogen sind, derart, dass der Oberflächenbereich des distalen Endes in Relation zu dem Befestigungszustand verringert ist, um eine Extraktion der medizinischen implantierbaren Leitung aus dem menschlichen oder tierischen Körper zu erleichtern.

## Revendications

1. Conducteur médical implantable, qui est adapté pour être fixé par une extrémité distale (2) à un tissu (3) à l'intérieur d'un corps humain ou animal, ledit conducteur comprenant une hélice rotative (5) à l'extrémité distale, destinée à permettre la fixation du conducteur au tissu en vissant l'hélice dans le tissu, et un manchon souple (6) à l'extrémité distale qui dépasse du reste du conducteur d'une certaine distance dans le sens axial, dans lequel le manchon souple (6) est formé avec des fentes axiales (8) à partir d'un segment annulaire non brisé (7) dans une zone située au niveau d'un bord distal le plus à l'extérieur du manchon et à une distance dans le sens proximal telle que des segments souples (9) sont formés dans le manchon, dans lequel, lorsqu'il se trouve dans une position voulue, une force agissant dans le sens axial sur le manchon souple entraîne la compression du manchon dans le sens axial en amenant les segments à se replier vers l'extérieur de façon à agrandir l'aire de surface de l'extrémité distale, **caractérisé en ce que** ledit manchon (6) comprend une formation de prise (10) sur le segment annulaire distal (7) telle que la formation de prise est en prise avec l'hélice et, lors du vissage de l'hélice dans le tissu, le manchon est comprimé par le déplacement du segment annulaire dans le sens proximal.

2. Conducteur médical implantable selon la revendication 1, **caractérisé en ce que** chaque segment souple (9) est formé sur une face interne, au niveau d'une partie intermédiaire du segment, avec une rainure circonférentielle (12) destinée à faire fonction de charnière afin de faciliter le repliement des segments.

3. Conducteur médical implantable selon la revendication 2, **caractérisé en ce que** chaque segment souple (9) est formé sur une face externe à chaque extrémité du segment avec une rainure circonférentielle (12) destinée à faire fonction de charnière afin de faciliter le repliement des segments.

4. Conducteur médical implantable selon la revendication 3, **caractérisé en ce que** la rainure circonférentielle (12) la plus à l'intérieur de chaque segment souple (9) est positionnée au niveau de l'extrémité distale du reste du conducteur.

5. Conducteur médical implantable selon l'une quelconque des revendications précédentes, **caractérisé en ce que** la formation de prise se présente sous la forme d'un pion (10) à l'intérieur du segment annulaire (7).

6. Conducteur médical implantable selon l'une quelconque des revendications 1 à 4, **caractérisé en ce que** la formation de prise (10) se présente sous la forme d'une ouverture dans un disque sur le segment annulaire (7).

7. Conducteur médical implantable selon l'une quelconque des revendications précédentes, **caractérisé en ce que** le manchon souple (6) est pourvu d'au moins quatre segments souples (9).

8. Conducteur médical implantable selon l'une quelconque des revendications précédentes, **caractérisé en ce qu'**il possède également un état d'extraction dans lequel les segments (9) repliés vers l'extérieur sont pliés dans le sens distal de façon à réduire l'aire de surface de l'extrémité distale par rapport à l'état monté afin de faciliter l'extraction du conducteur médical implantable du corps humain ou animal.
